(19) Europäisches Patentamt
European Patent Office
Office européen des brevets

(11) EP 0 946 140 B1

(12) **FASCICULE DE BREVET EUROPEEN**

(45) Date de publication et mention de la délivrance du brevet:
**20.03.2002 Bulletin 2002/12**

(21) Numéro de dépôt: **97952098.8**

(22) Date de dépôt: **18.12.1997**

(51) Int Cl.[7]: **A61K 7/48**

(86) Numéro de dépôt international:
**PCT/FR97/02343**

(87) Numéro de publication internationale:
**WO 98/27957 (02.07.1998 Gazette 1998/26)**

# (54) UTILISATION D'UN EXTRAIT DE CORDIA DICHOTOMA

VERWENDUNG VON CORDIA DICHOTOMA EXTRAKTEN

USE OF AN EXTRACT OF CORDIA DICHOTOMA

(84) Etats contractants désignés:
**DE ES FR GB IT**

(30) Priorité: **20.12.1996 FR 9615794**

(43) Date de publication de la demande:
**06.10.1999 Bulletin 1999/40**

(73) Titulaire: **PARFUMS CHRISTIAN DIOR**
**75008 Paris (FR)**

(72) Inventeurs:
- **RENIMEL, Isabelle**
  **F-45470 Trainou (FR)**
- **OLIVIER, Marc**
  **F-30113 Les Angles (FR)**
- **ANDRE, Patrice**
  **F-45170 Neuville aux Bois (FR)**
- **CABALION, Pierre**
  **F-98848 NourméaCedex (FR)**

(74) Mandataire: **Giraud, Françoise**
**Cabinet Beau de Loménie**
**158, rue de l'Université**
**75340 Paris Cedex 07 (FR)**

(56) Documents cités:
- **PATENT ABSTRACTS OF JAPAN vol. 96, no. 012 & JP 08 208498 A (SUNSTAR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 946 140 B1

Printed by Jouve, 75001 PARIS (FR)

## Description

L'invention concerne des utilisations d'un extrait de la plante Cordia dichotoma, dans les domaines cosmétique et pharmaceutique, notamment dermatologique.

La plante Cordia dichotoma est une plante de la famille des Boraginaceae que l'on trouve en particulier en Nouvelle Calédonie.

Cette plante est bien connue en médecine traditionnelle polynésienne pour ses applications comme anti-inflammatoire.

On prépare en particulier à partir de cette plante des cataplasmes.

Ses propriétés émollientes sont également bien connues ainsi que ses propriétés d'asepsie.

Les inventeurs de la présente demande ont maintenant découvert que des extraits de cette plante présentaient en outre d'excellentes propriétés anti-élastasique, ce qui permet leur utilisation comme principe actif dans des compositions cosmétiques ou pharmaceutiques, notamment dermatologiques, destinées à lutter contre toutes les manifestations liées au vieillissement de la peau.

Plus précisément, l'invention résulte d'essais systématiques réalisés par les inventeurs en vue d'étudier les réactions enzymatiques des extraits de plante Cordia dichotoma.

Ces essais systématiques ont permis de mettre en évidence une certaine activité de ses extraits sur l'élastase.

Plus précisément, les essais systématiques réalisés par les inventeurs ont porté sur les enzymes suivantes : élastase, tyrosinase et 3',5'AMPc-phosphodiestérase.

L'élastase, enzyme de dégradation de l'élastine, est présente dans les cellules, notamment dans les cellules dermiques (fibroblastes) ainsi que, dans une moindre mesure, dans les cellules de l'épiderme (kératinocytes). On a observé que la quantité et l'activité de l'élastase augmentent au cours du processus de vieillissement cutané, aussi bien intrinsèque qu'actinique. Par une dégradation des fibres d'élastine, l'action de l'élastase a pour conséquence une perte de l'élasticité cutanée, un relâchement de la peau et l'apparition de rides.

La 3',5'-AMPc-phosphodiestérase, désignée ci-après "phosphodiestérase" ou "PDE", est l'enzyme qui transforme l'AMPc, second messager qui intervient dans le contrôle du métabolisme cellulaire, en AMP inactif. L'inhibition de la PDE par un agent inhibiteur permet en conséquence de maintenir un taux intracellulaire élevé d'AMPc, ce qui a pour effet notamment d'activer les protéines kinases A, et, par ce processus, permet de favoriser la dégradation des lipides.

De plus, il est également connu que l'AMPc intervient pour contrer certains processus inflammatoires (M. Hitchcock, J. Immunol. (1977) 188 557). Egalement, il a été décrit que la phosphodiestérase augmente avec l'âge (S. K. Puri et L. Volicer, Mechanisms of Aging and Dev. (1981) 15 239). De ce fait, son inhibition contribuera à lutter contre les effets du vieillissement, en particulier au niveau cutané.

La tyrosinase est l'enzyme clé de la synthèse de la mélanine et donc du métabolisme de la pigmentation cutanée. En cosmétique, son inhibition, par des agents appropriés, trouve des applications dans le traitement local des hyperpigmentations cutanées, telles que les tâches pigmentaires de sénescence.

Ces tests ont montré la très nette activité d'extraits de la plante Cordia dichotoma sur l'inhibition de l'élastase, et ont conduit les inventeurs de la présente invention à la préparation de compositions cosmétiques ou dermatologiques utiles dans toutes les applications où l'on cherche à lutter contre le vieillissement de la peau.

Ainsi, selon l'une de ses caractéristiques essentielles, l'invention concerne l'utilisation d'un extrait de la plante Cordia dichotoma comme principe actif d'une composition cosmétique destinée à lutter contre les effets du vieillissement sur la peau, notamment en préservant ou améliorant les qualités biomécaniques de la peau, en particulier son élasticité, en retardant l'apparition des rides ou en diminuant leur profondeur, et en améliorant la fermeté cutanée.

Selon une autre caractéristique essentielle de l'invention, elle concerne également l'utilisation d'un extrait de la plante Cordia dichotoma pour la préparation d'une composition pharmaceutique, notamment dermatologique, destinée au traitement des effets du vieillissement intrinsèque ou actinique de la peau, ledit extrait étant incorporé dans un véhicule pharmaceutiquement acceptable.

Dans ces deux domaines d'application cosmétique et dermatologique, ce sont essentiellement les feuilles qui s'avèrent intéressantes pour la préparation des extraits de l'invention.

L'extrait est avantageusement obtenu par macération de la plante ou partie de plante dans un solvant polaire ou un mélange de solvants polaires, suivie d'une filtration. Le solvant de la solution obtenue peut être au besoin évaporé pour obtenir l'extrait sec.

De préférence, l'évaporation sera réalisée sous pression réduite.

A titre de solvants avantageusement utilisés, on citera :

- l'eau,
- les alcools en $C_1$ à $C_6$, tels que le méthanol, l'éthanol et l'isopropanol
- les polyols en $C_2$ à $C_6$, tels que le propylèneglycol ou le glycérol, et leurs mélanges.

L'eau s'avère être un solvant d'extraction particulièrement intéressant.

On pourra également obtenir l'extrait de la plante par la technique dite de l'extraction au dioxyde de carbone supercritique.

Selon un mode de réalisation avantageux, cette composition cosmétique ou pharmaceutique comprend de 0,001 à 10 % en poids et en particulier de 0,02 à 1 % en poids d'extrait sec de la plante par rapport au poids total de la composition finale.

Par ailleurs, les essais réalisés par les inventeurs ont clairement montré que, non seulement le rendement d'extraction était lié à la nature du solvant utilisé mais également l'activité enzymatique de l'extrait. Les exemples joints en annexe montrent clairement l'effet du choix du solvant sur l'activité enzymatique de l'extrait..

Les compositions cosmétiques ou pharmaceutiques selon l'invention peuvent être formulées selon toute forme acceptable pour leur emploi en cosmétologie ou en pharmacie, notamment en dermatologie. Il peut, en particulier, s'agir d'une composition sous forme appropriée pour une application topique, en particulier sous forme d'une crème ou d'un gel, en particulier d'une crème ou d'un gel pour le visage, les mains, le buste ou le corps.

Selon un autre aspect, l'invention concerne l'utilisation de l'extrait de plante comme agent cosmétique, ledit agent étant incorporé dans une composition cosmétique telle que définie précédemment.

Cet agent cosmétique sera notamment utilisé dans toutes les applications où l'on recherche, en particulier, à inhiber l'action de l'élastase.

Les compositions cosmétiques de l'invention seront également utilisées en particulier pour lutter contre les effets du vieillissement sur la peau, notamment en préservant ou améliorant les qualités biomécaniques de la peau, en particulier son élasticité, en retardant l'apparition des rides ou en diminuant leur profondeur et en améliorant la fermeté cutanée.

Ainsi, selon un autre aspect, l'invention concerne des compositions cosmétiques destinées au soin de la peau, en particulier pour lutter contre les effets du vieillissement cutané.

Comme on l'a vu précédemment, l'efficacité des compositions cosmétiques aussi bien que dermatologiques précédemment décrites a pu être corrélée avec l'activité anti-élastasique de l'extrait.

Dans toutes les applications, les compositions utilisées sont de préférence des compositions à application topique destinées à être appliquée sur la peau.

Les exemples qui suivent sont donnés à titre purement illustratif de l'invention.

Sauf indication contraire, les proportions données dans les exemples de compositions sont exprimées en pourcentage en poids

Exemple I

Préparation d'un extrait aqueux selon l'invention

1 g de feuilles de la plante Cordia dichotoma préalablement séchées et broyées est introduit dans 200 ml d'eau. La suspension est laissée, à température ambiante sous agitation modérée, pendant 4 heures. On filtre ensuite le mélange, puis on évapore le solvant du filtrat ainsi obtenu sous pression réduite. On récupère alors l'extrait sec. Pour préparer les compositions selon l'invention, il est possible d'utiliser soit la solution d'extrait de plante, éventuellement concentrée, soit l'extrait sec.

Exemple 2

Mise en évidence de l'activité inhibitrice des extraits sur différentes enzymes 2.1 Extrait utilisés

Les tests d'inhibition enzymatiques étant menés en milieu aqueux, il est nécessaire d'utiliser des solvants d'extraction miscibles avec l'eau.

Ainsi, en procédant comme décrit dans l'exemple 1, on a réalisé trois extraits différents avec respectivement de l'eau, du méthanol et du DMSO. La concentration de ces extraits est ensuite ajustée à 0,5 % en extrait sec de plante, soit par ajout soit par évaporation du solvant d'extraction.

Tous les tests décrits ci-après ont été réalisés en trois exemplaires. Les valeurs rapportées sont des moyennes arithmétiques.

2.2. Inhibition de l'élastase

a) Principe du test :

Les techniques de mise en évidence de l'inhibition de l'élastase ont été décrites par différents auteurs (BAUMSTARK, J.S. et al., Biochim. Biophys. Acta (1963), 77, 676 ; BIETH, B. et al., Biochem. Med., (1974), 11, 350 ; C. FRANCK, I. BYRJALSEN, Biol. Chem. Hoppe Seyler, (1988) 369 (8) 677-82).

Le principe actif est le suivant : un substrat est mis en présence d'élastase en milieu aqueux, puis, après incubation, on effectue une mesure des produits de la réaction.

En l'occurrence, le substrat est le N-succinyl-$(Ala)_3$-para-nitroanilide, disponible auprès de la Société Sigma (Réf. : S 4760) en solution à 0,5 mg/ml dans un tampon Tris-HCl 0,2 M à pH 8,8. L'élastase ajoutée au milieu réactionnel libère la para-nitroaniline et le résidu peptidique. L'évolution de la réaction est observée au spectrophotomètre Uvikon 941® (Kontron S.A.) à la longueur d'onde $\lambda$ de 379 nm.

La composition du milieu réactionnel est la suivante :

- solution de substrat (0,5 mg/ml de tampon) : 200 µl
- tampon Tris-HCl : 600 µl
- solvant d'extraction : 100 µl

Le solvant d'extraction contient ou non l'effecteur, c'est-à-dire l'extrait de la plante Cordia dichotoma, à la concentration de 0,5 % en poids, selon qu'il s'agit de l'essai avec effecteur, ou de l'essai sans effecteur (activité basale de l'enzyme).

A ces 900 µl de milieu réactionnel, on ajoute extemporanément 100 µl de la solution d'enzyme à raison de 35 U/ml dans le tampon Tris-HCl.

On mesure alors la cinétique de la libération de para-nitroaniline par l'absorption d'une lumière mono-chromatique de longueur d'onde 379 nm au moyen du spectrophotomètre, ce qui permet de calculer le pourcentage d'inhibition $I_E$ suivant la formule -ci-dessous :

$$I_E = \frac{\Delta AbB / mn - \Delta AbE / mn}{\Delta AbB / mn} \times 100$$

dans laquelle ΔAbB / mn est la différence d'absorbance par minute du milieu réactionnel pour l'activité basale, et ΔAbE / mn, la différence d'absorbance du milieu réactionnel pour l'essai avec effecteur. Les valeurs d'absorbance prises en compte sont celles correspondantes à la période linéaire de l'évolution de l'absorbance en fonction du temps.

Les résultats figurent au tableau I ci-après.

2.3 Inhibition de la phosphodiestérase (PDE)

Le principe de ce test est basé sur l'hydrolyse de la 3',5'-adénosine monophosphate cyclique ($AMP_c$) en adénosine monophosphate (AMP). La formation d'AMP est mesurée par analyse HPLC.

La composition du milieu réactionnel est indiquée ci-dessous. Les solutions des réactifs sont réalisées dans du tampon Tris-HCl 0,05 M à pH 7,5.

- solution d'$AMP_c$ (substrat) à 0,25% dans le tampon 80 µl
- solvant, sans ou avec effecteur à 0,5% 80 µl
- tampon Tris-HCl 480 µl

A ce milieu, on ajoute extemporanément 160 µl de PDE à 0,5 U/ml dans le tampon Tris-HCl.

Au temps t = 5 minutes, on mesure la quantité d'AMP formée en calculant la surface d'intégration du pic de l'AMP sur le chromatogramme fourni par l'appareil HPLC (KONTION S.A.).

On peut alors estimer le taux d'inhibition de la PDE $I_A$ par l'effecteur suivant la formule :

$$I_A = \frac{SAMP_b - SAMP_e}{SAMP_b} \text{ X } 100$$

dans laquelle $SAPP_b$ représente la surface d'intégration du pic de l'AMP pour l'activité basale de l'enzyme (sans effecteur) et $SAMP_e$ représente la surface d'intégration du pic de l'AMP pour l'activité de l'enzyme en présence de l'effecteur.

Les résultats obtenus figurent en Tableau I ci-après.

2.4 Inhibition de la tyrosinase

Le principe de ce test est basé sur la formation de dopachrome à partir de la L-tyrosine, par l'action de la tyrosinase.

Il est rappelé que la L-tyrosine, en présence de tyrosinase et d'oxygène, s'oxyde en L-DOPA qui s'oxyde à son tour, encore par l'action de la tyrosinase, en dopaquinone. Celle-ci se cyclise alors en cyclodopa, qui s'oxyde en dopachrome, précurseur de mélanines et qui absorbe la lumière à la longueur d'onde de 480nm.

$$\text{L-tyrosine} \xrightarrow{\text{tyrosinase}} \text{L-DOPA} \xrightarrow{\text{tyrosinase}} \text{dopachrome } (\lambda = 480\text{ nm}) \dashrightarrow \text{mélanines}$$

La formation de dopachrome peut donc être suivie par spectrophotométrie.

Pour cette méthode, on pourra se reporter en particulier aux publications de S.H. Pomerantz. Arch. Biochem. Biophys. (1974) 160 73-82, ou de J. Barber, J. Invest. Dermtaol. (1984) 83 145-149.

La composition du milieu réactionnel est indiquée ci-dessous. Les solutions des réactifs sont réalisées dans du tampon phosphate 0,02 M à pH 6,9.

- solution de L-tyrosine (1er substrat) à 1 mM dans le tampon 333 µl
- solution de L-DOPA (2ème substrat) à 1 mM dans le tampon 333 µl
- solvant, sans ou avec effecteur à 0,5% 333 µl

A ce milieu réactionnel, on ajoute extemporanément 33 µl de solution de tyrosinase à la concentration de 2400 U/ml dans le tampon phosphate.

On mesure alors la cinétique de la formation de dopachrome par l'absorption d'une lumière monochromatique de longueur d'onde 480nm au moyen d'un spectrophotomètre Uvikon 941 (KONTION S.A.), ce qui permet de calculer, pour la partie linéaire de l'évolution de l'absorbance en fonction du temps, le pourcentage d'inhibition IT de la tyrosinase suivant la formule ci-dessous :

$$I_T = \frac{\Delta\ AbB/mn - \Delta\ AbE/mn}{\Delta AbB/mn} X\ 100$$

dans laquelle ΔAbB/mn est la différence d'absorbance par minute du milieu réactionnel pour l'activité basale de l'enzyme (sans effecteur), et ΔAbE/mn, la différence d'absorbance du milieu réactionnel pour l'essai avec effecteur.

Les résultats figurent au Tableau I ci-après.

TABLEAU I

| TAUX D'INHIBITION ENZYMATIQUE POUR LES EXTRAITS DE L'INVENTION | | | |
|---|---|---|---|
| | EXTRAITS | | |
| | $E_{eau}$ | $E_{methanol}$ | $E_{DMSO}$ |
| $I_{E(élastase)}$ | 96 | 1 | 14 |
| $I_{T\ (tyrosinase)}$ | 1 | | |
| $I_{A(PDE)}$ | 2 | 1 | 2 |

$E_{eau}$ : extrait aqueux, à 0,5 %
$E_{méthanol}$ : extrait méthanolique, à 0,5 %
$E_{DMSO}$ : extrait au DMSO, à 0,5 %

Les résultats figurant au Tableau I ci-dessus démontrent l'intérêt des extraits selon l'invention, dans les domaines de la cosmétique et de la pharmacie, notamment en dermatologie, dès qu'il s'agit de bloquer, de diminuer l'importance ou de réguler l'action de certaines enzymes.

Plus précisement, il ressort clairement que l'action de l'extrait aqueux de Cordia dichotoma est particulièrement importante sur l'inhibition de l'élastase. Cependant, cet extrait ne présente aucune action d'inhibition de la tyrosinase ni de la phosphodiéstase. Les extraits méthanoliques et DMSO de cette plante ne semblent avoir aucun effet inhibiteur sur les autres enzymes testées.

De ce fait, les extraits de Cordia dichotoma, en particulier les extraits aqueux, peuvent être avantageusement utilisés pour les différentes applications citées plus haut découlant de l'inhibition de l'élastase, telles que le raffermissement cutané et la lutte contre les effets du vieillissement de la peau, tels que l'apparition des rides.

Exemple 3

Gel cosmétique anti-rides

- Extrait sec de l'exemple 1 0,05 g
- Carbomer 0,3 g
- Glycérine 3,0 g
- EDTA tétrasodique 0,05 g
- Eau florale d'Hammamelis 3,00 g
- Polyméthylméthacrylate 1,00 g
- Parfums, conservateurs, colorant, neutralisant qs
- Eau distillée qsp 100 g

Ce gel présente un effet anti-rides et apaisant.

Exemple 4

Crème anti-rides pour le visage

- Extrait sec selon l'exemple 1, 0,5 g
- Glycéryl stéarate + PEG 100 stéarate 5,0 g
- Alcool cétylique 1,0 g
- Alcool stéarylique 1,0 g
- Cire d'abeille 1,50 g
- Squalane 3,0 g
- Polyisobutène hydrogéné 4,0 g
- Octanoate de cétéaryle 1,50 g
- Tricaprylate/caprate de glycérol 3,0 g
- Diméthicone 1,0 g
- Gomme xanthane 0,2 g
- Carbomer 0,15 g
- Glycérine 2,0 g
- Neutralisant, conservateur, parfums, colorants qs
- Eau qsp 100 g

Exemple 5

Crème pour peau sensible

- Extrait sec de familles de Cordia dichotoma préparé selon le procédé de l'exemple 1, 0,2 g
- Méthylglucose sesquistéarate 3,0 g
- Cire d'abeille 3,0 g
- Alcool béhénique 3,0 g
- Octanoate d'octyle 5,0 g
- Huile minérale fluide 7,5 g
- Octanoate de cétostéaryle 5,0 g
- Glycérine 3,0 g
- Gomme xanthane 0,50 g
- Parfums 0,30 g
- Conservateur, colorants qs
- Eau qsp 100 g

Cette crème est utilisée pour le raffermissement de la peau du visage et du cou.

**Revendications**

1. Utilisation, dans une composition cosmétique, d'un extrait de la plante Cordia dichotoma, en tant que principe actif destiné à lutter contre les effets du vieillissement sur la peau, notamment en préservant ou améliorant les qualités biomécaniques de la peau, en particulier son élasticité, en retardant l'apparition des rides ou en diminuant leur profondeur, et en améliorant la fermeté cutanée.

2. Utilisation d'un extrait de la plante Cordia dichotoma pour la préparation d'une composition pharmaceutique, notamment dermatologique, destinée au traitement des effets du vieillissement intrinsèque ou actinique de la peau, ledit extrait étant incorporé dans un véhicule pharmaceutiquement acceptable.

3. Utilisation selon la revendication 1 ou 2, **caractérisée en ce que** ledit extrait contenu dans ladite composition présente une action inhibitrice de l'élastase.

4. Utilisation selon l'une des revendications 1 à 3, **caractérisée en ce que** ledit extrait de ladite plante est un extrait de feuilles.

5. Utilisation selon l'une des revendications 1 à 4, **caractérisée en ce que** ledit extrait est obtenu par macération de ladite plante ou d'une partie de ladite plante dans un solvant polaire ou un mélange de solvants polaires, suivie d'une filtration et, au besoin, d'une évaporation du solvant.

6. Utilisation selon la revendication 5, caractérisée en ce ledit solvant est choisi dans le groupe constitué par l'eau, les alcools en $C_1$ à $C_6$, tels que le méthanol, l'éthanol et l'isopropanol, les polyols en $C_2$ à $C_6$, tels que le propylèneglycol et le glycérol, et leurs mélanges.

7. Utilisation selon l'une des revendications 4 à 6, **caractérisée en ce que** ledit solvant est l'eau.

8. Utilisation selon l'une des revendications 1 à 4, **caractérisée en ce que** ledit extrait est obtenu par extraction au dioxyde de carbone supercritique.

9. Utilisation selon l'une des revendications 1 à 8, caractérisée en ce ladite composition comprend de 0,001 % à 10 % en poids, et en particulier de 0,02 % à 1 % en poids, d'extrait sec de ladite plante.

10. Utilisation selon l'une des revendications 1 à 9, **caractérisée en ce que** ladite composition se présente sous toute forme appropriée pour une application topique, en particulier sous forme d'une crème ou d'un gel, en particulier d'une crème ou d'un gel pour le visage, les mains, le buste ou le corps.

## Patentansprüche

1. Verwendung eines Extraktes der Pflanze Cordia dichotoma in einer kosmetischen Zusammensetzung als Wirkprinzip, das dazu bestimmt ist, die Alterungseffekte auf die Haut zu bekämpfen, insbesondere indem die biomechanischen Eigenschaften der Haut konserviert oder verbessert werden, insbesondere deren Elastizität, durch Verzögern des Auftretens von Falten oder durch Verminderung deren Tiefe und durch Verbessern der Hautfestigkeit.

2. Verwendung eines Extraktes der Pflanze Cordia dichotoma zur Herstellung einer pharmazeutischen, insbesondere einer dermatologischen Zusammensetzung, welche zur Behandlung der Wirkung der intrinsischen oder aktinischen Alterung der Haut bestimmt ist, wobei das Extrakt in einem pharmazeutisch annehmbaren Träger aufgenommen ist.

3. Verwendung nach Anspruch 1 oder 2, **dadurch gekennzeichnet, dass** das in der Zusammensetzung enthaltene Extrakt eine Elastase inhibierende Wirkung zeigt.

4. Verwendung nach einem der Ansprüche 1 bis 3, **dadurch gekennzeichnet, dass** das Extrakt der Pflanze ein Blätterextrakt ist.

5. Verwendung nach einem der Ansprüche 1 bis 4, **dadurch gekennzeichnet, dass** das Extrakt durch Mazerierung der Pflanze oder eines Teils der Pflanze in einem polaren Lösungsmittel oder einem polaren Lösungsmittelgemisch erhalten wird, mit einer nachfolgenden Filtration und gegebenenfalls einem Verdampfen des Lösungsmittels.

6. Verwendung nach Anspruch 5, **dadurch gekennzeichnet, dass** das Lösungsmittel aus der Gruppe ausgewählt wird, die durch Wasser, die $C_1$- bis $C_6$-Alkohole, wie Methanol, Ethanol und Isopropanol, die $C_2$- bis $C_6$-Polyole, wie Propylenglycol oder Glycerin und deren Mischungen gebildet ist.

7. Verwendung nach einem der Ansprüche 4 bis 6, **dadurch gekennzeichnet, dass** das Lösungsmittel Wasser ist.

8. Verwendung nach einem der Ansprüche 1 bis 4, **dadurch gekennzeichnet, dass** das Extrakt durch Extraktion mit überkritischem Kohlendioxid erhalten ist.

9. Verwendung nach einem der Ansprüche 1 bis 8, **dadurch gekennzeichnet, dass** die Zusammensetzung 0,001 bis 10 Gew.-% und insbesondere 0,02 bis 1 Gew.-% Trockenextrakt der Pflanze umfasst.

10. Verwendung nach einem der Ansprüche 1 bis 9, **dadurch gekennzeichnet, dass** sich die Zusammensetzung in allen für eine topische Anwendung geeigneten Formen zeigt, insbesondere in Form einer Creme oder eines Geles, insbesondere einer Creme oder eines Geles für das Gesicht, die Hände, das Dekolleté oder den Körper.

## Claims

1. The use, in a cosmetic composition, of an extract of the plant *Cordia dichotoma* as active principle intended to combat the effects of ageing on the skin, especially by preserving or improving the biomechanical properties of the skin, particularly its elasticity, by delaying the appearance of wrinkles or by reducing their depth, and by improving the firmness of the skin.

2. The use of an extract of the plant *Cordia dichotoma* for the preparation of a pharmaceutical, especially dermatological composition intended for the treatment of the effects of intrinsic or actinic skin ageing, said extract being incorporated in a pharmaceutically acceptable vehicle.

3. The use according to claim 1 or 2, **characterized in that** said extract contained in said composition has an inhibitory action on elastase.

4. The use according to one of claims 1 to 3, **characterized in that** said extract of said plant is a leaf extract.

5. The use according to one of claims 1 to 4, **characterized in that** said extract is obtained by maceration of said plant or a part of said plant in a polar solvent or polar solvent mixture, followed by filtration and, if necessary,

evaporation of the solvent.

6. The use according to claim 5, **characterized in that** said solvent is selected from the group consisting of water, $C_1$ to $C_6$ alcohols such as methanol, ethanol and isopropanol, $C_2$ to $C_6$ polyols such as propylene glycol and glycerol, and mixtures thereof.

7. The use according to one of claims 4 to 6, **characterized in that** said solvent is water.

8. The use according to one of claims 1 to 4, **characterized in that** said extract is obtained by supercritical carbon dioxide extraction.

9. The use according to one of claims 1 to 8, **characterized in that** said composition comprises from 0.001% to 10% by weight and particularly from 0.02% to 1% by weight of dry extract of said plant.

10. The use according to one of claims 1 to 9, **characterized in that** said composition is presented in any form appropriate for topical application, in particular in the form of a cream or gel and particularly a cream or gel for the face, hands, bust or body.